(19) Europäisches Patentamt
European Patent Office
Office européen des brevets

(11) EP 1 190 705 A1

(12) EUROPEAN PATENT APPLICATION
published in accordance with Art. 158(3) EPC

(43) Date of publication:
**27.03.2002 Bulletin 2002/13**

(21) Application number: **00939146.7**

(22) Date of filing: **23.06.2000**

(51) Int Cl.[7]: **A61K 9/127**

(86) International application number:
**PCT/JP00/04140**

(87) International publication number:
**WO 01/00173 (04.01.2001 Gazette 2001/01)**

(84) Designated Contracting States:
**AT BE CH CY DE DK ES FI FR GB GR IE IT LI LU MC NL PT SE**
Designated Extension States:
**AL LT LV MK RO SI**

(30) Priority: **24.06.1999 JP 17814299**

(71) Applicant: **KYOWA HAKKO KOGYO CO., LTD.**
**Chiyoda-ku, Tokyo 100-8185 (JP)**

(72) Inventors:
- **KATO, Yasuki, Pharmaceutical Res. Inst.**
  **Sunto-gun, Shizuoka 411-8731 (JP)**
- **YAMAUCHI, Masahiro**
  **Sunto-gun, Shizuoka 411-8731 (JP)**
- **KUSANO, Hiroko**
  **Sunto-gun, Shizuoka 411-8731 (JP)**
- **ISHIHARA, Atsushi**
  **Sunto-gun, Shizuoka 411-8731 (JP)**

(74) Representative: **Casalonga, Axel et al**
**BUREAU D.A. CASALONGA - JOSSE**
**Morassistrasse 8**
**80469 München (DE)**

# (54) METHOD OF REGULATING LEAKAGE OF DRUG ENCAPSULATED IN LIPOSOMES

(57) The present invention provides a method of inhibiting the leakage of a drug encapsulated in liposomes, which comprises satisfying at least two requirements selected from the group consisting of the following three requirements: using at least two lipid bilayers of the liposomes, controlling the average particle size of the liposomes to 120 nm or more, and using lipid having a phase transition temperature higher than *in vivo* temperature as lipid constituting the liposomes. Also, the present invention provides a liposome preparation which is stable *in vivo* and satisfies at least two requirements selected from the group consisting of the following three requirements: the number of lipid bilayers of the liposomes is at least two, the liposomes have an average particle size of 120 nm or more, and lipid constituting the liposomes has a phase transition temperature higher than *in vivo* temperature.

EP 1 190 705 A1

## Description

### TECHNICAL FIELD

The present invention relates to a method of inhibiting the leakage of a drug encapsulated in liposomes and liposome preparations which are stable *in vivo.*

### BACKGROUND ART

It has already been a practice in the medical field to encapsulate drugs in liposomes and thus enhance the drug effects. The technique has been clinically applied mainly by the injection method. In intravascular administration among injection operations, it is important for enhancing the therapeutic effect that a drug encapsulated in liposomes remains in the liposomes over a relatively long period of time without leakage.

B. has found a method of inhibiting the leakage of an antitumor agent from liposomes (Japanese Patent No. 2,572,554). According to the method, a transmembrane potential is generated by providing a concentration gradient of a charged substance inside and outside of liposomes and a drug which can be ionized is encapsulated in the liposomes due to a pH gradient or a $Na^+/K^+$ concentration gradient to thereby inhibit the leakage of a drug from the liposomes. As a method of encapsulating a drug in liposomes and inhibiting the leakage thereof similarly using a pH gradient, Barenholz *et al.* have invented a method using a pH gradient inside and outside of liposomes which is achieved by an ammonium ion gradient using ammonium sulfate (Japanese Patent No. 2,659,136). Both of these methods are not restricted in the particle size of the liposomes to be used, and these liposomes involve small unilamellar vesicles (SUVs), large unilamellar vesicles (LUVs), multilamellar vesicles (MLVs) and the like. On the other hand, Maurer *et al.* reported that when ciprofloxacin was encapsulated in LUVs of 190 nm in an average particle size by the method under a pH gradient using ammonium sulfate, ciprofloxacin rapidly leaked out of the LUVs in 50% mouse serum at 37°C (*Biochim. Biophys. Acta,* 1374, 9 (1998)). According to this report, ciprofloxacin was not crystallized (precipitated) in the liposomes, different from doxorubicin or the like, and thus leaked out. Thus, the methods presented by the two patents as described above are not necessarily the most desirable methods from the viewpoint of the leakage of drugs encapsulated in liposomes. Therefore, further improvement has been required.

### DISCLOSURE OF THE INVENTION

An object of the present invention is to provide a method of inhibiting the leakage of a drug encapsulated in liposomes, and liposome preparations which are stable *in vivo.*

The inventors previously found that liposome preparations in which an indolocarbazole derivative, such as UCN-01 or the like, is encapsulated have improved stability and the like *in vivo* (WO97/48398).

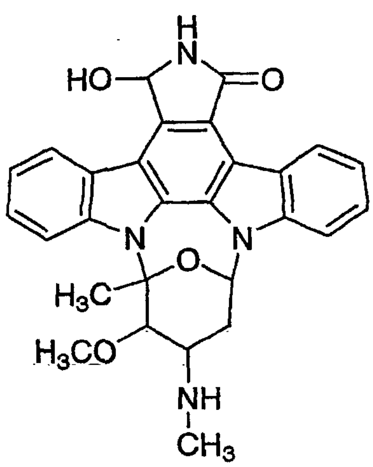

UCN-01

As the results of subsequent studies, the inventors have found that the leakage of a drug can be efficiently inhibited by controlling the average particle size of liposomes to 120 nm or more or using at least two lipid bilayers of the liposomes. Furthermore, they have found that the leakage of a drug can be inhibited by using a component having

a phase transition temperature higher than *in vivo* temperature as a component constituting the lipid bilayers.

Specifically, the present invention relates to a method of inhibiting the leakage of a drug encapsulated in liposomes in the presence of a biological component, which comprises using at least two lipid bilayers of the liposomes, or a method of inhibiting the leakage of a drug encapsulated in liposomes in the presence of a biological component, which comprises using lipid having a phase transition temperature higher than *in vivo* temperature as lipid constituting the liposomes.

Furthermore, the present invention relates to a method of inhibiting the leakage of a drug encapsulated in liposomes in the presence of a biological component, which comprises satisfying at least two requirements selected from the group consisting of the following three requirements: using at least two lipid bilayers of the liposomes, controlling the average particle size of the liposomes to 120 nm or more, and using lipid having a phase transition temperature higher than *in vivo* temperature as lipid constituting the liposomes.

Moreover, the present invention relates to a method of inhibiting the leakage of a drug encapsulated in liposomes in the presence of a biological component, which comprises using at least two lipid bilayers of the liposomes, and controlling the average particle size of the liposomes to 120 nm or more.

Also, the present invention provides a liposome preparation in which the number of lipid bilayers of the liposomes is at least two, and the liposomes have an average particle size of 120 nm or more, a liposome preparation in which the number of lipid bilayers of the liposomes is at least two, and lipid constituting the liposomes has a phase transition temperature higher than *in* vivo temperature, or a liposome preparation in which the liposomes have an average particle size of 120 nm or more, and lipid constituting the liposomes has a phase transition temperature higher than *in vivo* temperature.

Furthermore, the present invention provides a liposome preparation which satisfies at least two requirements selected from the group consisting of the following three requirements: the number of lipid bilayers of the liposomes is at least two, the liposomes have an average particle size of 120 nm or more, and lipid constituting the liposomes has a phase transition temperature higher than *in vivo* temperature.

Each of the liposome preparations as described above can inhibit the leakage of a drug encapsulated in liposomes in the presence of a biological component.

Examples of the lipid constituting the liposomes include phospholipid, glyceroglycolipid, sphingoglycolipid, cholesterol, and the like. Particularly, phospholipid is preferably used. Among these, it is preferable to use lipid having a phase transition temperature higher than *in vivo* temperature (35 to 37°C). The lipid may be modified by a nonionic surfactant such as polysorbate 80, Pluronic *F68, etc.;* a cationic surfactant such as benzalkonium chloride *etc.*; an anionic surfactant such as sodium laurylsulfate *etc.*; a polysaccharide such as dextran *etc.*, or a derivative thereof; a polyoxyethylene derivative such as polyoxyethylene lauryl alcohol, polyethylene glycol, *etc.*; or the like.

Examples of the phospholipid include natural or synthetic phospholipids, such as phosphatidylcholine (soybean phosphatidylcholine, yolk phosphatidylcholine, distearoyl phosphatidylcholine, dipalmitoyl phosphatidylcholine, *etc.*), phosphatidylethanolamine (distearoyl phosphatidylethanolamine, dipalmitoyl phosphatidylethanolamine, *etc.*), phosphatidylserine, phosphatidic acid, phosphatidylglycerol, phosphatidylinositol, lysophosphatidylcholine, sphingomyelin, polyethylene glycol-modified phospholipid, yolk lecithin, soybean lecithin, hydrogenated phospholipid, *etc.*; and the like. Among these, it is preferable to use phospholipid having a phase transition temperature higher than in *vivo* temperature (35 to 37°C) (for example, distearoyl phosphatidylcholine, dipalmitoyl phosphatidylethanolamine, *N*-stearoyl sphingomyelin, *etc.*)

Examples of the glyceroglycolipid include sulfoxyribosylglyceride, diglycosyldiglyceride, digalactosyldiglyceride, galactosyldiglyceride, glycosyldiglyceride, and the like. Among these, it is preferable to use glyceroglycolipid having a phase transition temperature higher than *in vivo* temperature (35 to 37°C) (for example, 1,2-*O*-dipalmitoyl-3-*O*-β-D-glucuronosyl-sn-glycerol, 1,2-*O*-distearoyl-3-*O*-β-D-glucuronosyl-sn-glycerol, *etc.*)

Examples of the sphingoglycolipid include galactosylcerebroside, lactosylcerebroside, ganglioside, and the like. Among these, it is preferable to use sphingoglycolipid having a phase transition temperature higher than *in vivo* temperature (35 to 37°C) (for example, *N*-stearoyldihydrogalactosylsphingosine, *N*-stearoyldihydrolactosylsphingosine, *etc.*)

These lipids may be used alone or in combination. When the lipids are used in combination, lipid comprising at least two components selected from hydrogenated soybean phosphatidylcholine, polyethylene glycol-modified phospholipid and cholesterol, lipid comprising at least two components selected from distearoyl phosphatidylcholine, polyethylene glycol-modified phospholipid and cholesterol, or the like is used as the lipid. As the phospholipid in the polyethylene glycol-modified phospholipid as described herein, phosphatidylethanolamine, such as distearoyl phosphatidylethanolamine or the like, is preferably used.

If necessary, it is possible to use, together with the lipid component, a membrane-stabilizing agent, for example, a sterol such as cholesterol *etc.*; an antioxidant such as tocopherol *etc.*; a charged substance such as stearylamine, dicetyl phosphate, ganglioside, *etc.*

Examples of the drug to be encapsulated in liposomes include indolocarbazole derivatives, an antitumor

agent, an antibiotic, an antifungal agent, a pharmaceutically active substance, and the like.

Examples of the indolocarbazole derivatives include UCN-01, derivatives thereof (for example, the following compounds), and the like:

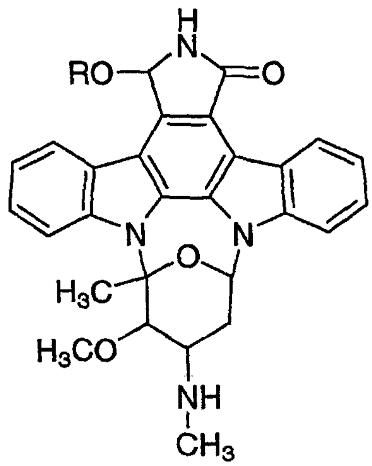

wherein R represents hydrogen or lower alkyl.

The lower alkyl in the definition of R means linear or branched alkyl having 1 to 6 carbon atoms such as methyl, ethyl, propyl, isopropyl, *sec*-butyl, *tert*-butyl, pentyl, hexyl, or the like.

Examples of the antitumor agent include actinomycin D, mitomycin C, chromomycin, doxorubicin, epirubicin, vinorelbine, daunorubicin, aclarubicin, bleomycin, peplomycin, vincristine, vinblastine, vindesine, etoposide, methotrexate, 5-Fu, tegafur, cytarabine, enocitabine, ancitabine, taxol, taxotere, cisplatin, cytosine arabinoside, irinotecan, derivatives thereof, and the like.

Examples of the antibiotic include minocycline, tetracycline, piperacillin sodium, sultamicillin tosylate, amoxicilline, ampicillin, bacampicillin, aspocicilin, cefdinir, flomoxef sodium, cefotiam, cefcapene pivoxil, cefaclor, ceftoren pivocil, cephazolin sodium, cefozoran, clarithromycin, clindamycin, erythromycin, levofloxacin, tosufloxacin tosylate, ofloxacin, ciprofloxacin, arbekacin, isepamicin, dibekacin, amikacin, gentamicin, vancomycin, fosfomycin, derivatives thereof, and the like.

Examples of the antifungal agent include fluconazole, itraconazole, terbinafine, amphotericin B, miconazole, derivatives thereof, and the like.

Examples of the pharmaceutically active substance include a hormone, an enzyme, a protein, a peptide, an amino acid, a nucleic acid, a gene, a vitamin, a saccharide, lipid, a synthetic drug, and the like.

Examples of the biological component include a blood component and the like.

Next, a method of producing the liposome preparations according to the present invention will be described.

The liposome preparations of the present invention can be produced by using known methods for producing liposome preparations. Examples of these known methods for producing liposome preparations include a method of preparing liposomes reported by Bangham *et al. (J. Mol. Biol.*, 13, 238 (1965)), an ethanol injection method (*J. Cell. Biol.*, 66, 621 (1975)), a French press method (*FEBS Lett.,* 99, 210 (1979)), a freezing and thawing method (*Arch. Biochem. Biophys.*, 212, 186 (1981)), a reversed phase evaporation method (*Proc. Natl. Acad. Sci. USA*, 75, 4194 (1978)), a pH gradient method (Japanese Patent No. 2,572,554, Japanese Patent No. 2,659,136, *etc.)),* and the like.

The pH gradient method has a number of advantages such that a high drug-encapsulation ratio in liposomes can be achieved, and that little organic solvent remains in the liposome suspension. For example, the lipid is dissolved in a solvent such as ethanol or the like, the resultant mixture is placed into a round bottomed flask, and the solvent is evaporated under reduced pressure to thereby form a thin lipid film. Then, an acidic buffer (for example, citrate buffer) is added thereto, followed by shaking, to thereby form large MLVs. Next, the average particle size of the liposomes is controlled to the desired level (for example, 130 nm) by an extrusion method or the like. After a weakly acidic solution of a drug such as UCN-01 or the like is added to the liposome suspension, a suitable pH regulator (e.g., aqueous sodium hydroxide) is added thereto to raise the pH of the liposome suspension to around the neutral pH (the difference between the pH of the liposome suspension before and after the rise of pH is preferably 3 or more). By the above operation, the drug can be quantitatively encapsulated in the liposomes.

If necessary, it is also possible to modify the surface of the liposomes using a nonionic surfactant, a cationic surfactant, an anionic surfactant, a polysaccharide or a derivative thereof, a polyoxyethylene derivative, or the like *(Stealth Liposomes,* ed. by D.D. Lasic and F. Martin, CRC Press Inc., Florida, pp. 93-102, 1995). For the application

to targeting, it is also possible to modify the surface of the liposomes with an antibody, a protein, a peptide, a fatty acid, or the like *(Stealth Liposomes,* ed. by D.D. Lasic and F. Martin, CRC Press Inc., Florida, pp. 93-102, 1995).

In addition to water, examples of the solution in which the liposomes are suspended include an acid, an alkali, various buffers, physiological saline, an amino acid infusion, and the like. Furthermore, an antioxidant such as citric acid, ascorbic acid, cysteine, ethylenediaminetetraacetic acid (EDTA), or the like, or an isotonic agent such as glycerol, glucose, sodium chloride, or the like, may be added to the liposome suspension.

Alternatively, liposomes can be formed by dissolving a drug and lipid in an organic solvent such as ethanol or the like, evaporating the solvent, and then adding physiological saline or the like thereto, followed by shaking under stirring.

The average particle size of the liposomes is preferably 120 nm or more, more preferably 120 to 500 nm. The average particle size can be controlled by, for example, the extrusion method as mentioned above.

Examples of a method of providing at least two lipid bilayers of the liposomes include the extrusion method using a membrane filter having relatively large pores (0.2 μm, 0.4 μm or above), a method of mechanically grinding large MLVs (using a Manton-Gorlin, a micro-fluidizer, or the like) (ed. and written by R.H. Muller, S. Benita and B. Bohm, "Emulsion and Nanosuspensions for the Formulation of Poorly Soluble Drugs", *High-Pressure Homogenization Techniques for the Production of Liposome Dispersions:* Potential and Limitations, M. Brandl, pp. 267-294, 1998 (Scientific Publishers Stuttgart, Germany)), and the like.

The liposome preparation obtained by the above method or the like can be used as such. Alternatively, it may be mixed with a filler such as mannitol, lactose, glycine, or the like, and then freeze-dried, depending on the purpose of use, storage conditions, or the like. It is also possible to add a freeze-drying agent, such as glycerine or the like, thereto, followed by freeze-drying.

Although the liposome preparations obtained by the present invention are generally used as an injection, these may also be used as an oral preparation, a nasal preparation, an eye drop, a percutaneous preparation, a suppository, an inhalant, or the like by manufacturing the preparation into such forms.

The liposome preparations obtained by the present invention are prepared in order to stabilize a drug in a biological component (for example, a blood component), to reduce side effects and to increase accumulation in tumors.

Next, the effects of the present invention will be described by reference to the following Test Example.

Test Example 1

In order to monitor the leakage of UCN-01 encapsulated in liposomes in human AGP-containing rat plasma (human AGP: 0.5 mg/mL) with the lapse of time, 0.1 mL of the UCN-01-containing liposome suspensions prepared in Examples 1 to 4 and Comparative Example 1 to 3 were each mixed with 0.9 mL of distilled water. To 0.05 mL of the resultant mixture, 4.95 mL of the rat plasma containing 0.5 mg/mL human AGP was added and mixed to obtain a liquid sample. Immediately after mixing, and after storing at 37°C for 3 hours, 2 mL of the liquid sample was subjected to gel filtration (Sepharose CL-6B, 20 mm in diameter × 20 cm, mobile phase: PBS (phosphate-buffered saline), amount of sample added: 2 mL, fraction collection amount: about 4 mL). After separating the liposome fraction from the protein fraction, 0.8 mL of 2-propanol was added per 0.4 mL of the eluate, followed by shaking. Then, the resultant mixture was centrifuged (12,000 x g, 10 minutes) at 4°C, and 20 μl of the supernatant was analyzed by high performance liquid chromatography (HPLC) under the following conditions.

HPLC analysis conditions:

Column:

YMC-Pack ODS-AM AM-312 150 mm × 6 mm (YMC)

Mobile phase:

A 0.1% triethylamine-containing 0.05 mol/L
phosphate buffer (pH 7.3) : acetonitrile = 1:1 (parts by volume)

Flow rate:

1.0 mL/min

Column retention temperature:

25°C

Detection:

Excitation wavelength 310 nm, fluorescence wavelength 410 nm

The remaining ratio of UCN-01 in liposomes was calculated in accordance with the following equation by determining the UCN-01 content in the liposome fraction and then correcting it with the use of the recovery (i.e., the sum of UCN-1 in the liposome fraction and the protein fraction) in the gel filtration ((A+B)/C):

$$\text{UCN-01 content (\%) in liposome fraction} = (A/C) \times 100$$

$$\text{UCN-01 content (\%) in protein fraction} = (B/C) \times 100$$

A: the amount of UCN-01 contained in the liposome fraction.
B: the amount of UCN-01 contained in the protein fraction.
C: the amount of UCN-01 contained in the liposome suspension subjected to gel filtration.

$$\text{Remaining ratio (\%) of UCN-01 in liposomes}$$

$$= (\text{UCN-01 content (\%) in liposome fraction/recovery}$$

$$\text{(\%) in gel filtration}) \times 100$$

The results are shown in Table 1.

Table 1:

| Remaining ratio (%) of UCN-01 in liposomes | | |
|---|---|---|
| | | UCN-01 remaining ratio (%) |
| Example 1 | Immediately after mixing | 95 |
| | After 3 hours | 80 |
| Example 2 | Immediately after mixing | 91 |
| | After 3 hours | 57 |
| Example 3 | Immediately after mixing | 94 |
| | After 3 hours | 63 |
| Example 4 | Immediately after mixing | 99 |
| | After 3 hours | 81 |
| Comparative Example 1 | Immediately after mixing | 90 |
| | After 3 hours | 37 |
| Comparative Example 2 | Immediately after mixing | 23 |
| | After 3 hours | 0 |

Table 1: (continued)

| Remaining ratio (%) of UCN-01 in liposomes | | |
|---|---|---|
| | | UCN-01 remaining ratio (%) |
| Comparative Example 3 | Immediately after mixing | 93 |
| | After 3 hours | 5 |

Next, Examples and Comparative Examples of the present invention will be given.

MODE FOR CARRYING OUT THE INVENTION

Example 1

To 5 g of hydrogenated soybean phosphatidylcholine {phase transition temperature: 58°C (*FEBS Lett.*, 386, 247-251 (1996))} was added 25 mL of a 100 mmol/L citrate buffer (pH 4.0), followed by shaking under stirring with a vortex mixer. The suspension was passed through a polycarbonate membrane filter (0.4 µm) 10 times at 70°C. Then, a 100 mmol/L citrate buffer was added thereto to give a liposome suspension having a concentration of hydrogenated soybean phosphatidylcholine of 62.5 mg/mL. Separately, 10 mg of UCN-01 was taken and 8 mL of the liposome suspension prepared above was added thereto. The pH of the resultant mixture was adjusted to 8 by adding an appropriate amount of 1 mol/L aqueous sodium hydroxide, and then distilled water was added thereto to give a total volume of 10 mL. The mixture was heated at 70°C for 5 minutes to thereby encapsulate UCN-01 in liposomes.
The average particle size of the liposomes measured by the dynamic light scattering (DLS) method (A model DLS-700, Otsuka Electronics Ltd.; the same applies hereinafter) was 186 nm.

Example 2

To 5 g of hydrogenated soybean phosphatidylcholine {phase transition temperature: 58°C (*FEBS Lett.,* 386, 247-251 (1996))} was added 25 mL of a 100 mmol/L citrate buffer (pH 4.0), followed by shaking under stirring with a vortex mixer. The suspension was passed through a polycarbonate membrane filter (0.4 µm) twice at 70°C, and further passed through a polycarbonate membrane filter (0.2 µm) 10 times at 70°C. Then, a 100 mmol/L citrate buffer was added thereto to give a liposome suspension having a concentration of hydrogenated soybean phosphatidylcholine of 62.5 mg/mL. Separately, 10 mg of UCN-01 was taken and 8 mL of the liposome suspension prepared above was added thereto. The pH of the resultant mixture was adjusted to 8 by adding an appropriate amount of 1 mol/L aqueous sodium hydroxide. Then, distilled water was added thereto to give a total volume of 10 mL. The mixture was heated at 70°C for 5 minutes to thereby encapsulate UCN-01 in liposomes.
The average particle size of the liposomes measured by the DLS method was 130 nm.

Example 3

To 5 mL of the liposome suspension containing UCN-01 as prepared in Example 2 was added 0.05 mL of a 1.25 g/mL solution of PEG-DSPE {1,2-distearoyl-sn-glycero-3-phosphatidylethanolamine-*N*-(polyethylene glycol 2000); manufactured by Avanti} in ethanol. Then, the mixture was heated at 70°C for 2 minutes to thereby coat the surface of the liposomes with polyethylene glycol (PEG).
The average particle size of the liposomes measured by the DLS method was 136 nm.

Example 4

To 0.7 g of distearoyl phosphatidylcholine [DSPC, phase transition temperature: 58°C and 56°C (ed. by Shoshichi Nojima *et al., Liposome,* p.77, 1988, Nankodo)] was added about 5 mL of a 100 mmol/L citrate buffer (pH 4.0), followed by shaking under stirring with a vortex mixer. The suspension was passed through a polycarbonate membrane filter (0.4 µm) 10 times at 70°C, and further passed through a polycarbonate membrane filter (0.2 µm) 10 times at 70°C. Then, a 100 mmol/L citrate buffer was added thereto to give a liposome suspension having a DSPC concentration of 62.5 mg/mL. Separately, 5 mg of UCN-01 was taken and 4 mL of the liposome suspension prepared above was added thereto. The pH of the resultant mixture was adjusted to 8 by adding an appropriate amount of 1 mol/L aqueous sodium hydroxide. Then, distilled water was added thereto to give a total volume of 5 mL. The mixture was heated at 70°C for 5 minutes to thereby encapsulate UCN-01 in liposomes.
The average particle size of the liposomes measured by the DLS method was 180 nm.

Comparative Example 1

To 20 g of hydrogenated soybean phosphatidylcholine {phase transition temperature: 58°C (*FEBS Lett.*, 386, 247-251 (1996))} was added 70 mL of a 100 mmol/L citrate buffer (pH 4.0), followed by shaking under stirring with a vortex mixer. The suspension was passed through a polycarbonate membrane filter (0.4 μm) 4 times at 70°C, and further passed through a polycarbonate membrane filter (0.1 μm) 10 times at 70°C. Then, a 100 mmol/L citrate buffer was added thereto to give a liposome suspension having a concentration of hydrogenated soybean phosphatidylcholine of 62.5 mg/mL. Separately, 20 mg of UCN-01 was taken and 16 mL of the liposome suspension prepared above was added thereto. The pH of the resultant mixture was adjusted to 8 by adding an appropriate amount of 1 mol/L aqueous sodium hydroxide. Then, distilled water was added thereto to give a total volume of 20 mL. The mixture was heated at 70°C for 5 minutes to thereby encapsulate UCN-01 in liposomes. After ice-cooling, 1.6 mL of the liposome suspension containing UCN-01 was taken and 6.4 mL of distilled water was added thereto. The resultant mixture was ultracentrifuged (25°C, 110,000 g × 1 hour), and 6.7 mL of the supernatant was removed. Then, distilled water was added to the precipitate, followed by re-suspending to give a UCN-01 concentration of 1 mg/mL.
The average particle size of the liposomes measured by the DLS method was 109 nm.

Comparative Example 2

To 15 g of yolk phosphatidylcholine [EggPC, phase transition temperature: -15 to -7°C (ed. by Shoshichi Nojima *et al.*, *Liposome*, p.77, 1988, Nankodo)] was added 75 mL of a 100 mmol/L citrate buffer (pH 4.0), followed by shaking under stirring with a vortex mixer. The suspension was passed through a polycarbonate membrane filter (0.4 μm) 10 times at room temperature. Then, a 100 mmol/L citrate buffer was added thereto to give a liposome suspension having an EggPC concentration of 62.5 mg/mL. Separately, 5 mg of UCN-01 was taken and 4 mL of the liposome suspension prepared above was added thereto. The pH of the resultant mixture was adjusted to 8 by adding an appropriate amount of 1 mol/L aqueous sodium hydroxide. Then, distilled water was added thereto to give a total volume of 5 mL. UCN-01 was encapsulated in liposomes at room temperature.
The average particle size of the liposomes measured by the DLS method was 274 nm.

Comparative Example 3

To 1.1 g of dipalmitoyl phosphatidylcholine [DPPC, phase transition temperature: 41°C and 35°C (ed. by Shoshichi Nojima *et al., Liposome,* p.77, 1988, Nankodo)] was added about 7 mL of a 100 mmol/L citrate buffer (pH 4.0), followed by shaking under stirring with a vortex mixer. The suspension was passed through a polycarbonate membrane filter (0.4 μm) 15 times at 55°C, and further passed through a polycarbonate membrane filter (0.2 μm) 10 times at 55°C. Then, a 100 mmol/L citrate buffer was added thereto to give a liposome suspension having a DPPC concentration of 62.5 mg/mL. Separately, 5 mg of UCN-01 was taken, and 4 mL of the liposome suspension prepared above was added thereto. The pH of the resultant mixture was adjusted to 8 by adding an appropriate amount of 1 mol/L aqueous sodium hydroxide. Then, distilled water was added thereto to give a total volume of 5 mL. UCN-01 was encapsulated in liposomes by heating the mixture at 55°C for 5 minutes.
The average particle size of the liposomes measured by the DLS method was 179 nm.

INDUSTRIAL APPLICABILITY

The present invention provides a method of inhibiting the leakage of a drug encapsulated in liposomes and a liposome preparation which is stable *in vivo.*

**Claims**

1. A method of inhibiting the leakage of a drug encapsulated in liposomes in the presence of a biological component, which comprises using at least two lipid bilayers of the liposomes.

2. A method of inhibiting the leakage of a drug encapsulated in liposomes in the presence of a biological component, which comprises using lipid having a phase transition temperature higher than *in vivo* temperature as lipid constituting the liposomes.

3. A method of inhibiting the leakage of a drug encapsulated in liposomes in the presence of a biological component, which comprises satisfying at least two requirements selected from the group consisting of the following three

requirements: using at least two lipid bilayers of the liposomes, controlling the average particle size of the liposomes to 120 nm or more, and using lipid having a phase transition temperature higher than *in vivo* temperature as lipid constituting the liposomes.

**4.** The method of inhibiting the leakage according to claim 2 or 3, wherein the lipid comprises at least one component selected from the group consisting of hydrogenated soybean phosphatidylcholine, polyethylene glycol-modified phospholipid, and cholesterol.

**5.** The method of inhibiting the leakage according to claim 2 or 3, wherein the lipid comprises at least one component selected from the group consisting of distearoyl phosphatidylcholine, polyethylene glycol-modified phospholipid, and cholesterol.

**6.** A method of inhibiting the leakage of a drug encapsulated in liposomes in the presence of a biological component, which comprises using at least two lipid bilayers of the liposomes, and controlling the average particle size of the liposomes to 120 nm or more.

**7.** The method of inhibiting the leakage according to claim 3 or 6, wherein the liposomes have an average particle size of 120 to 500 nm.

**8.** The method of inhibiting the leakage according to any one of claims 1 to 7, wherein the biological component is a blood component.

**9.** The method of inhibiting the leakage according to any one of claims 1 to 8, wherein the drug encapsulated is an indolocarbazole derivative.

**10.** The method of inhibiting the leakage according to any one of claims 1 to 8, wherein the drug encapsulated is an antitumor agent.

**11.** The method of inhibiting the leakage according to any one of claims 1 to 8, wherein the drug encapsulated is an antibiotic.

**12.** The method of inhibiting the leakage according to any one of claims 1 to 8, wherein the drug encapsulated is a pharmaceutically active substance.

**13.** A liposome preparation in which the number of lipid bilayers of the liposomes is at least two, and the liposomes have an average particle size of 120 nm or more.

**14.** A liposome preparation in which the number of lipid bilayers of the liposomes is at least two, and lipid constituting the liposomes has a phase transition temperature higher than *in vivo* temperature.

**15.** A liposome preparation in which the liposomes have an average particle size of 120 nm or more, and lipid constituting the liposomes has a phase transition temperature higher than *in vivo* temperature.

**16.** A liposome preparation which satisfies at least two requirements selected from the group consisting of the following three requirements: the number of lipid bilayers of the liposomes is at least two, the liposomes have an average particle size of 120 nm or more, and lipid constituting the liposomes has a phase transition temperature higher than *in vivo* temperature.

**17.** The liposome preparation according to any one of claims 13 to 16, which inhibits the leakage of a drug encapsulated in the liposomes in the presence of a biological component.

**18.** The liposome preparation according to claim 17, wherein the biological component is a blood component.

**19.** The liposome preparation according to any one of claims 14 to 18, wherein the lipid comprises at least one component selected from the group consisting of hydrogenated soybean phosphatidylcholine, polyethylene glycol-modified phospholipid, and cholesterol.

**20.** The liposome preparation according to any one of claims 14 to 18, wherein the lipid comprises at least one component selected from the group consisting of distearoyl phosphatidylcholine, polyethylene glycol-modified phospholipid, and cholesterol.

**21.** The liposome preparation according to any one of claims 13 and 15 to 18, wherein the liposomes have an average particle size of 120 to 500 nm.

**22.** The liposome preparation according to any one of claims 13 to 21, wherein the drug encapsulated is an indolocarbazole derivative.

**23.** The liposome preparation according to any one of claims 13 to 21, wherein the drug encapsulated is an antitumor agent.

**24.** The liposome preparation according to any one of claims 13 to 21, wherein the drug encapsulated is an antibiotic.

**25.** The liposome preparation according to any one of claims 13 to 21, wherein the drug encapsulated is a pharmaceutically active substance.

## INTERNATIONAL SEARCH REPORT

International application No.
PCT/JP00/04140

A. CLASSIFICATION OF SUBJECT MATTER
Int.Cl$^7$ A61K9/127

According to International Patent Classification (IPC) or to both national classification and IPC

B. FIELDS SEARCHED

Minimum documentation searched (classification system followed by classification symbols)
Int.Cl$^7$ A61K9/127

Documentation searched other than minimum documentation to the extent that such documents are included in the fields searched

Electronic data base consulted during the international search (name of data base and, where practicable, search terms used)
CAPLUS (STN)

C. DOCUMENTS CONSIDERED TO BE RELEVANT

| Category* | Citation of document, with indication, where appropriate, of the relevant passages | Relevant to claim No. |
|---|---|---|
| X<br><br>Y | WO, 97/03652, A1 (DEPOTECH CORPORATION), 06 February, 1997 (06.02.97), Full text & JP, 11-508900, A & US, 5931809, A | 1<br><br>3-14,16-25 |
| X<br><br>Y | JP, 8-59503, A (Teijin Limited), 05 March, 1996 (05.03.96), Full text (Family: none) | 1<br><br>3-14, 16-25 |
| X<br><br>Y | EP, 451791, A2 (HOECHST AKTIENGESELLSCHAFT), 16 October, 1991 (16.10.91), Full text & JP, 4-234820, A | 2<br><br>3-12,14-25 |
| Y | JP, 2-86841, A (TERUMO CORORATION), 27 March, 1990 (27.03.90), page 2, lower right column (Family: none) | 3-13,15-25 |
| Y | JP, 7-41432, A (Teijin Limited), 10 February, 1995 (10.02.95), Par. No. [0020] (Family: none) | 3-13,15-25 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 September, 2000 (08.09.00) | 19 September, 2000 (19.09.00) |
| Name and mailing address of the ISA/<br>Japanese Patent Office<br><br>Facsimile No. | Authorized officer<br><br>Telephone No. |

Form PCT/ISA/210 (second sheet) (July 1992)

**INTERNATIONAL SEARCH REPORT**

International application No.
PCT/JP00/04140

C (Continuation). DOCUMENTS CONSIDERED TO BE RELEVANT

| Category* | Citation of document, with indication, where appropriate, of the relevant passages | Relevant to claim No. |
|---|---|---|
| Y | EP, 850646, A1 (KYOWA HAKKO KOGYO CO., LTD.), 01 July, 1998 (01.07.98), Full text & WO, 97/48398, A1 | 9,22 |

Form PCT/ISA/210 (continuation of second sheet) (July 1992)